# EUROPEAN PATENT APPLICATION

(11) **EP 2 286 809 A1**
(43) Date of publication of application: **23.02.2011**
(21) Application number: 09735775.0
(22) Date of filing: 09.04.2009
(51) Int. Cl.: A61K 31/405, A61K 31/203, A61P 17/00

(54) **PERSONALISED PHARMACEUTICAL COMPOSITION CONTAINING RETINOIC ACID, FOR ANTI-AGING OF THE SKIN**

(30) Priority: 23.04.2008 ES 200801253
(71) Applicant: Umbert Millet, Ignacio, 08017 Barcelona (ES)
(72) Inventor: Umbert Millet, Ignacio, 08017 Barcelona (ES)
(74) Representative: Ponti Sales, Adelaida
(86) International application number: PCT/ES2009/000206
(87) International publication number: WO 2009/130344

(57) **Abstract**

The invention relates to a personalised pharmaceutical composition for anti-aging of the skin, in the form of an ointment, comprising a first group of common active principles present in any formulation, formed by retinoic acid, at least one anti-inflammatory agent, at least one additional depigmenting agent, at least one anti-oxidant and at least one vitamin; and a second group of variable active principles present in variable quantities formulated according to the metabolic characteristics of the particular user. The anti-inflammatory agent is indomethacin. The depigmenting agents can be selected from the group consisting of hydroquinone, kojic acid, mequinol, phytic acid and alpha-arbutin. The anti-oxidants can be selected from the group consisting of lipoic acid, lycopene, coenzyme Q10, resveratrol, pycnogenol®, L-carnosine, taurine, N-acetylglucosamine, ascorbic acid, isoflavones and tocotrienol. The second group of variable active principles can comprise hyaluronic acid, aloe vera, bilberry glycolic extract, centella glycolic extract, allantoin, organic silicon, niacinamide and a cicatrizant.

## Description

### TECHNICAL FIELD OF THE INVENTION

The present invention relates to a pharmaceutical composition for skin rejuvenation ("anti-aging"), of the kind that contains retinoic acid, at least one depigmenting agent and at least one anti-inflammatory agent.

### BACKGROUND OF THE INVENTION.

For many years now, retinoic acid, or acid forms of vitamin A, has been used to treat a variety of skin conditions, such as aging, acne, wrinkles, psoriasis, age spots and discoloration. See, for example, 1) Vahlquist, A. et al., J. Invest. zermatol., Vol. 94, Holland D. B. and Cunliffe, W. J. (1990), pg. 496-498; Ellis, C. N. et al., "Pharmacology of Retinols in Skin", Vasel, Karger, Vol. 3, (1989), pg. 249-252; Lowe, N. J. et al., "Pharmacology of Retinols in Skin", Vol. 3, (1989), pg. 240-248; 2) Fourie, Stephanus Petrus "'Dermatological preparation"'. CHEMICAL ABSTRACTS, vol. 90, no. 12, 19 March 1979, Columbus, Ohio, US; abstract no. 92433; 3) Kligman, AM, "Guidelines for the use of topical tretinoin (Retin-A) for photoaged skin", J Am. Acad. Dermatol. 1989; 21:650-4; 4) Klgman et al., "Topical tretionoin for photoaged skin", J Am. Acad. Dermatol. 1986; 15:836-59. Patents WO93/19743, EP625045, GB-A906000, FR2785185 and EP836476 disclose dermatologic and cosmetic uses of forms of retinoic acid, also recognized as tretinoin. The latter two disclose formulations containing retinoic acid and EP836476 discloses a cosmetic formulation also containing an anti-inflammatory substance.

Therefore, EP836476 defines a composition according to the preamble of claim 1. Since hydroquinone is a contraindicated and even banned substance in some countries, hydroquinone should be replaced by a technical equivalent thereof, i.e. concerning its antipigmenting activity.

However, the preceding formulations are not without drawbacks, consisting principally in that its therapeutic effect is highly variable and unpredictable. According to the present inventors, the variability is largely due to metabolic differences between patients.

The present invention is intended to provide a formulation of a drug substance and not merely a cosmetic substance, to solve the aforementioned problem and that is equally effective regardless of the particularities of the subject patients.

### EXPLANATION OF THE INVENTION

For this purpose, the object of the invention is a new-concept pharmaceutical composition adapted to the patient, for skin rejuvenation, of the type previously indicated, and that is essentially and primarily characterized, according to claim 1, in that it comprises two groups of active ingredients:
- A first group of common active ingredients present in any formulation, composed of said retinoic acid, said anti-inflammatory agent, one or more additional depigmenting agents, one or more antioxidants and one or more vitamins; and
- A second group of variable active ingredients, which are involved in varying amounts formulated on the basis of the metabolic characteristics of the particular user.

In particular, it is envisaged that such anti-inflammatory agent should be indomethacin.

Claim 2 and subsequent claims disclose embodiments of the pharmaceutical composition of the present invention.

In particular, the depigmenting agents are selected from the group formed by of hydroquinone, kojic acid, mequinol, phytic acid and alpha-arbutin.

Antioxidants may be selected from the group formed by lipoic acid, green tea EGCG catechins, lycopene, coenzyme Q 10, resveratrol, pycnogenol ®, L-carnosine, taurine, ascorbic acid, N-acetylglucosamine, isoflavones and tocotrienol.

Preferably, the vitamins are selected from the group formed by:
Vitamin E acetate, and Vitamin C.
In a preferred embodiment, the first group of common active ingredients comprise:
between 0.010 and 1% by weight of retinoic acid;
between 1 and 4% by weight of indomethacin;
between 1 and 6% hydroquinone;
between 1 and 5% Kojic acid;
between 1 and 3% mequinol;
between 1 and 6% lipoic acid;
between 2 and 6% vitamin E acetate, and
between 1 and 6% vitamin C.

This second group of variable active ingredients comprises: between 0.1 and 1.5% by weight of hyaluronic acid; up to 15% by weight of aloe vera; between 1 and 5% bilberry glycolic extract; between 1 and 5% centella glycolic extract; up to 2% by weight of allantoin; up to 15% by weight of organic silicon; varying proportions of niacinamide, vitamin F; a cicatrizant agent; at least one hormone; at least topical antibiotic and at least one coenzyme for energy production.

The niacinamide may be present in a proportion of 4 to 6% by weight.

Vitamin F may be present in a proportion of 0.1 to 4% by weight.

The cicatrizant agent is for instance rosehip oil, present in a proportion of between 2 and 6% by weight.

Preferably, hormones are selected from the group formed by: oestrogen and progesterone, in a proportion of between 1 and 3% by weight.

The antibiotics are preferably non-antagonistic antibiotics selected from the group formed by sodium sulfacetamide, clindamycin, ciprofloxacin and erythromycin.

Preferably, coenzymes are selected from the group formed by Co10 and NADH.

In addition, the second group of variable active ingredients may comprise a preservative antioxidant, such as sodium bisulfite.

This second group of variable active agents may include moisturizers, such as ammonium lactate or aloe vera.

It also discloses a personalized pharmaceutical composition for skin rejuvenation, comprising suitable excipients to be formulated as a topical ointment.

It also discloses a process for skin rejuvenation with the aforementioned personalized pharmaceutical composition and ointment comprising the following sequential steps:
- A controlled inflammation of the treated tissue is produced;
- Subsequently, a biostimulation of the treated tissue is produced, and
- Dermal and epidermal tissue is regenerated;
with the resulting rejuvenation of the dermal and epidermal tissue treated.

### BRIEF DESCRIPTION OF THE DRAWINGS

The following is a detailed description of preferred embodiments - although not limited to them - of the pharmaceutical composition of the invention, for whose better understanding drawings are attached wherein:
Figure 1 is a view of an area of aging skin in the area of the cheekbone and eye bags, with pronounced wrinkles and discoloration of the skin of a patient before applying the pharmaceutical composition of the invention;
Figure 2 is a view of the same skin area shown in Fig 1, but after treatment with an ointment of the present invention;

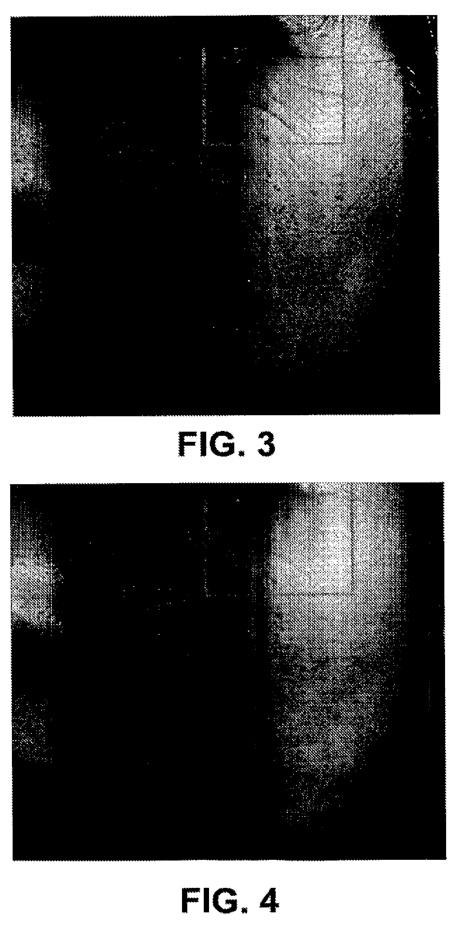

Figure 3 is a view of an area of aging skin of the cheekbone area, the left eye bag and the cheek, with pronounced wrinkles on an aging skin of another patient, before applying the pharmaceutical composition of the invention;
Figure 4 is a view of the same skin area shown in Fig 3, but after treatment with an ointment of the present invention;

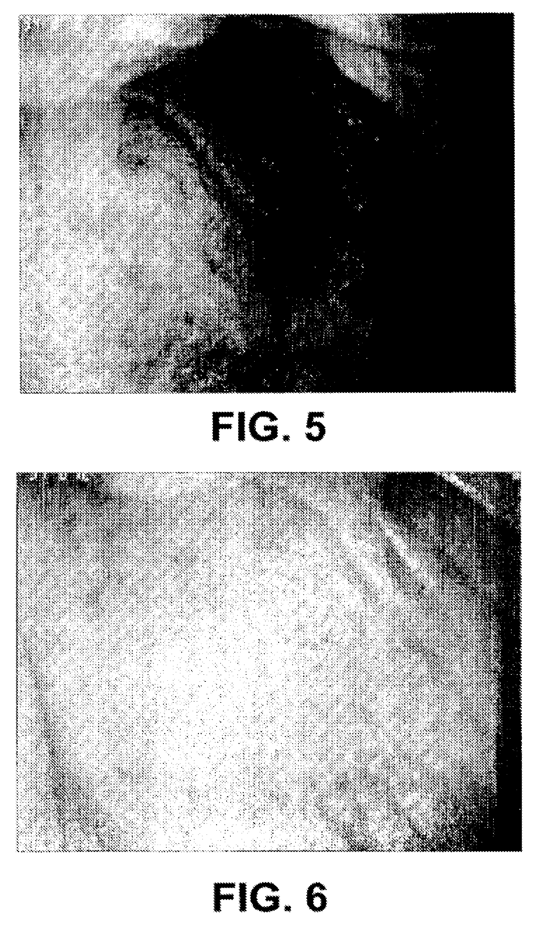

Figure 5 is a view of an area of aging skin of a cheek, a large age spot and epidermis wrinkles before applying the pharmaceutical composition of the invention;
Figure 6 is a view of the same skin area shown in Fig 5, but after treatment with an ointment of the present invention;

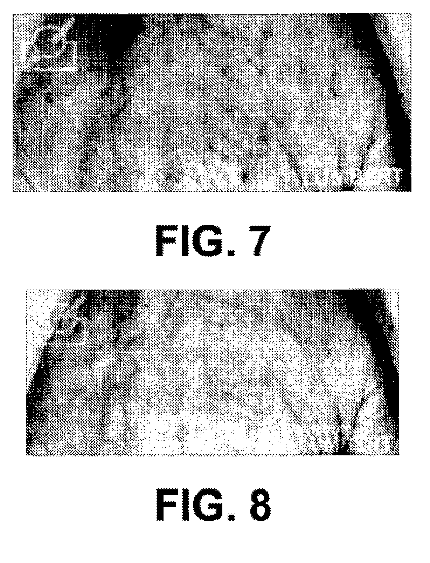

Figure 7 is a last view of an area of aging skin on the hand of another patient, with multiple spots and wrinkles before applying the pharmaceutical composition of the invention;
Figure 8 is a view of the same skin area shown in Fig 7, but after treatment with an ointment of the present invention;

### DETAILED DESCRIPTION OF PREFERRED EMBODIMENTS

The object of the invention is a drug product that, preferably in the pharmacological form of a topical ointment, is used in dermatology for aging prevention and anti-aging treatment. The main characteristic of the formulation of the present invention is that the active ingredients are divided into two groups: a first group of common active ingredients present in any formulation, and a second group of variable active ingredients, which are possibly involved in varying amounts formulated depending on the metabolic characteristics of the particular user.

Therefore, because it is an ad hoc drug, the formulation can only be prescribed by a medical practitioner after a careful study of the subject's metabolism.

The first group of common active ingredients present in any formulation of this anti-aging drug, comprises retinoic acid, one or more anti-inflammatory agents, one or more additional depigmenting agents, one or more antioxidants and one or more vitamins;

The depigmenting agents may be, for example, and not limited to: hydroquinone, kojic acid, mequinol, phytic acid and alpha-arbutin.

Said vitamins can be, for example and not limited to: Vitamin E acetate, and Vitamin C.

Antioxidants may be, for example and not limited to, one or more of the following: lipoic acid, green tea catechins (EGCG), lycopene, coenzyme Q 10, resveratrol, pycnogenol ®, L-carnosine, N-acetylglucosamine, taurine, isoflavones and tocotrienol. It should be noted that Coenzyme Q 10 is cited as an enzyme but may also have an antioxidant action. These active substances may act alone or combined with vitamins (e.g. lycopene with vitamin C and E).

The inventors have found that for the first group of common active ingredients the preferred compositions are:
between 0.010 and 1% by weight of retinoic acid;
between 1 and 4% by weight of indomethacin;
between 1 and 6% hydroquinone;
between 1 and 5% Kojic acid;
between 1 and 3% mequinol;
between 1 and 6% lipoic acid;
between 2 and 6% vitamin E acetate, and
between 1 and 6% vitamin C.

The second group of variable active ingredients may comprise one, several or all of the following active ingredients:
between 0.1 and 1.5% by weight of hyaluronic acid;
   up to 15% by weight of aloe vera;
between 1 and 5% bilberry glycolic extract;
between 1 and 5% centella glycolic extract;
   up to 2% by weight of allantoin;
   up to 15% by weight of organic silicon;
between 4 and 6% by weight of niacinamide (nicotinamide); between 0.1 to 4% by weight of vitamin F;
   a cicatrizant agent;
   at least one hormone;
   at least one topical antibiotic, and
   at least one coenzyme for energy production.

The preferred cicatrizant is rosehip oil

Hormones are selected primarily - though not exclusively - depending on the sex of the patient, from the group formed by: oestrogen and progesterone, and may be present in an amount between 1 and 3% by weight. It is important to note at this point that potentially cancer causing oestrogen should be avoided.

The antibiotics are non-antagonistic antibiotics preferably selected from the group formed by of sodium sulfacetamide, clindamycin, ciprofloxacin and erythromycin.

The coenzyme is selected from the group formed by Co10 and NADH.

The second group of variable active ingredients may comprise a preservative antioxidant, such as sodium bisulfite.

In addition, the personalized pharmaceutical composition for skin rejuvenation, according to the present invention will comprise suitable excipients so as to be formulated as a topical ointment.

Those skilled in the art will understand that, given the association and the proportion of the active ingredients involved in this formulation, a possible combined mechanism reaction, whereby according to the invention the anti-aging effect for skin is achieved, is as follows:
1. firstly a controlled inflammation of the treated tissue is produced;
2. Subsequently, biostimulation;
3. followed by regeneration of dermal and epidermal tissue;
4. which produces the skin rejuvenation.

### Example.

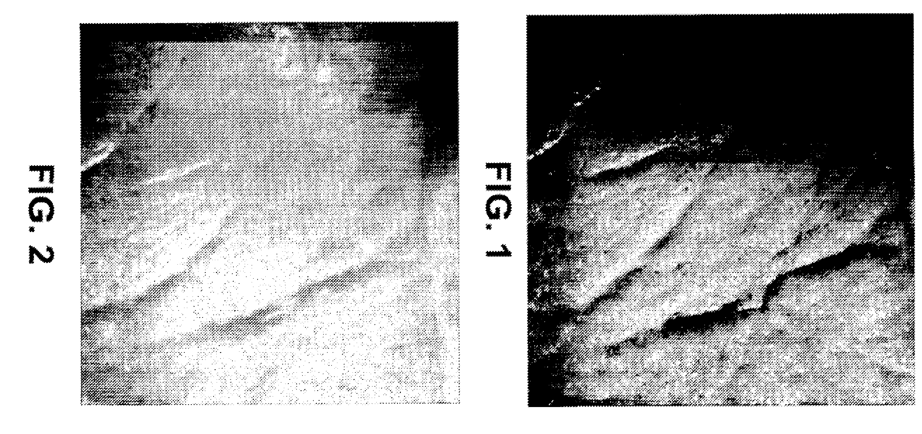

After a patient's metabolic study (Fig. 1), an ointment was prepared with the following percentage proportions (by weight) of active ingredients:
First group: common active ingredients
   0.01, Retinoic acid;
   3, indomethacin;
   5, hydroquinone;
   3, kojic acid;
   2, mequinol;
   4, lipoic acid;
   5, vitamin E acetate; and
   3, vitamin C.
Second group: variable active ingredients
   0.5, hyaluronic acid;
   5, aloe vera;
   2, bilberry glycolic extract;
   3, centella glycolic extract;
   0.2, allantoin;
   5, organic silicon;
   5, niacinamide (nicotinamide);
   1, vitamin F;
   5, rosehip oil;
   2, progesterone
   2, N-Acetylglucosamine, and
   11, Ammonium lactate

### Specific antioxidants

4, green tea
0,.3 coenzyme Q10
1, resveratrol
1, pycnogenol
2, L-carnosine
0.5, Taurine
5, isoflavones
0.5, lycopene

0.05% by weight of sodium bisulfite and qs for 50 g of ointment, "Beeler" based as an excipient, were added.

The ointment was administered topically to a patient with an aging cheekbone, illustrated in Figure 1.

During the interval the proportions of retinoic acid were changed "ceteris paribus" (0.010%, 0.025%, 0.050%, 0.1%, 0.2% and 0.3% proportions by weight) depending on the evolution of the results, always according to a doctor.

After applying said ointment for 15 months, the condition lessened remarkably and surprisingly, until showing a very improved appearance, as shown in Figure 2, with a significant rejuvenating effect, perceptible in the decrease in depth and number of wrinkles and in skin colour. Thereafter, aging did not revert to the appearance shown initially, yet occurred as a normal process in accordance with the patient's life.

Those skilled in the art will understand that the compounds mentioned herein can be replaced or supplemented by their technical equivalents. For example, sulfacetamide sodium, clindamycin, ciprofloxacin and erythromycin may be replaced with antibiotic or antibiotics with an equivalent action, remaining within the scope of the invention as claimed.

## Claims

1. A personalized pharmaceutical composition for skin rejuvenation, of the kind that contain retinoic acid, and at least one anti-inflammatory agent, **characterized in that** it comprises a first group of common active ingredients present in any formulation, formed by said retinoic acid, said at least one anti-inflammatory agent, one or more additional depigmenting agents, one or more antioxidants and one or more vitamins; and a second group of variable active ingredients, involved in varying amounts formulated on the basis of the metabolic characteristics of the particular user, said anti-inflammatory agent being indomethacin.

2. A personalized pharmaceutical composition for skin rejuvenation, according to claim 1, **characterized in that** said depigmenting agents are selected from the group formed by hydroquinone, kojic acid, mequinol, phytic acid and alpha-arbutin.

3. A personalized pharmaceutical composition for skin rejuvenation, according to claim 1, **characterized in that** said antioxidant is selected from the group formed by lipoic acid, lycopene, coenzyme Q 10, resveratrol, pycnogenol ®, L-carnosine, taurine, N-acetylglucosamine, ascorbic acid, isoflavones and tocotrienol.

4. A personalized pharmaceutical composition for skin rejuvenation, according to claim 1, **characterized in that** said vitamins are selected from the group formed by vitamin E acetate, and Vitamin C.

5. A personalized pharmaceutical composition for skin rejuvenation, according to claim 1, **characterized in that** said first group of common active ingredients comprise:
between 0.010 and 1% by weight of retinoic acid;
between 1 and 4% by weight of indomethacin;
between 1 and 6% hydroquinone;
between 1 and 5% Kojic acid;
between 1 and 3% mequinol;
between 1 and 6% lipoic acid;
between 2 and 6% vitamin E acetate, and
between 1 and 6% vitamin C.

6. A personalized pharmaceutical composition for skin rejuvenation, according to claim 5, **characterized in that** the second group of variable active ingredients comprise hyaluronic acid.

7. A personalized pharmaceutical composition for skin rejuvenation, according to claim 6, **characterized in that** said hyaluronic acid is present in an amount between 0.1 and 1.5% by weight.

8. A personalized pharmaceutical composition for skin rejuvenation, according to claim 1, **characterized in that** said second group of variable active ingredients comprises up to 15% by weight of aloe vera.

9. A personalized pharmaceutical composition for skin rejuvenation, according to claim 1, **characterized in that** said second group of variable active ingredients comprises between 1 and 5% of bilberry glycolic extract.

10. A personalized pharmaceutical composition for skin rejuvenation, according to claim 1, **characterized in that** said second group of variable active ingredients comprises between 1 and 5% of Centella glycolic extract.

11. A personalized pharmaceutical composition for skin rejuvenation, according to claim 1, **characterized in that** said second group of variable active ingredients comprises up to 2% by weight of allantoin.

12. A personalized pharmaceutical composition for skin rejuvenation, according to claim 1, **characterized in that** said second group of variable active ingredients comprises up to 15% by weight of organic silicon.

13. A personalized pharmaceutical composition for skin rejuvenation, according to claim 1, **characterized in that** said second group of variable active ingredients comprises niacinamide.

14. A personalized pharmaceutical composition for skin rejuvenation, according to claim 13, **characterized in that** niacinamide is present in a proportion of 4 to 6% by weight.

15. A personalized pharmaceutical composition for skin rejuvenation, according to claim 1, **characterized in that** said second group of variable active ingredients comprises vitamin F.

16. A personalized pharmaceutical composition for skin rejuvenation, according to claim 15, **characterized in that** said vitamin F is present in a proportion of 0.1 to 4% by weight.

17. A personalized pharmaceutical composition for skin rejuvenation, according to claim 1, **characterized in that** said second group of variable active ingredients comprises a cicatrizant.

18. A personalized pharmaceutical composition for skin rejuvenation, according to claim 17, **characterized in that** said cicatrizant is rosehip oil.

19. A personalized pharmaceutical composition for skin rejuvenation, according to claim 18, **characterized in that** said rosehip oil is present in a proportion of between 2 and 6% by weight.

20. A personalized pharmaceutical composition for skin rejuvenation, according to claim 1, **characterized in that** said second group of variable active ingredients comprises one or more hormones.

21. A personalized pharmaceutical composition for skin rejuvenation, according to claim 20, **characterized in that** said hormones are selected from the group formed by oestrogen and progesterone, in a proportion of between 1 and 3% by weight.

22. A personalized pharmaceutical composition for skin rejuvenation, according to claim 1, **characterized in that** said second group of variable active ingredients comprises one or more topical antibiotics.

23. A personalized pharmaceutical composition for skin rejuvenation, according to claim 22, **characterized in that** said antibiotics are non-antagonistic antibiotics selected from the group formed by: sodium sulfacetamide, clindamycin, ciprofloxacin and erythromycin.

24. A personalized pharmaceutical composition for skin rejuvenation, according to claim 1, **characterized in that** said second group of variable active ingredients comprises at least one coenzyme for energy production.

25. A personalized pharmaceutical composition for skin rejuvenation, according to claim 1, **characterized in that** said coenzyme is selected from the group formed by Co10 and NADH

26. A personalized pharmaceutical composition for skin rejuvenation, according to claim 1, **characterized in that** said second group of variable active ingredients comprises a preservative antioxidant, such as sodium bisulphite.

27. A personalized pharmaceutical composition for skin rejuvenation, according to claim 1, **characterized in that** said second group of variable active ingredients comprises a moisturizing agent such as ammonium lactate

28. A personalized pharmaceutical composition for skin rejuvenation, according to any of the preceding claims, **characterized in that** it comprises suitable excipients to be formulated as a topical ointment.

29. A process for skin rejuvenation with a personalized pharmaceutical composition according to any of the preceding claims, **characterized in that** it comprises the following sequential steps:
- A controlled inflammation of the treated tissue is produced;
- Subsequently, biostimulation of the treated tissue is produced, and
- The dermal and epidermal tissue is regenerated;
with the resulting rejuvenation of the dermal and epidermal tissue treated.
